# EUROPÄISCHE PATENTSCHRIFT

(11) **EP 0 662 470 B1**
(45) Veröffentlichungstag und Bekanntmachung des Hinweises auf die Patenterteilung: **22.01.1997**
(21) Anmeldenummer: 94117726.3
(22) Anmeldetag: 10.11.1994
(51) Int. Cl.: C07C 231/02, C07C 233/58

(54) **Verfahren zur Herstellung von Cyclopropancarbonsäureamid**
Process for the preparation of cyclopropane-carboxylic-amide
Procédé pour la préparation de cyclopropanamide

(30) Priorität: 07.01.1994 DE 4400328
(43) Veröffentlichungstag der Anmeldung: 12.07.1995
(73) Patentinhaber: HÜLS AKTIENGESELLSCHAFT, 45764 Marl (DE)
(72) Erfinder: Kleemiss, Wolfgang, Dr., D-45721 Haltern (DE)

(56) Entgegenhaltungen:
- EP-A- 0 043 949
- EP-A- 0 205 403
- EP-A- 0 365 970
- DE-A- 1 939 759
- FR-A- 2 093 472

## Beschreibung

Die vorliegende Erfindung betrifft ein neues Verfahren zur Herstellung von Cyclopropancarbonsäureamid durch Amidierung eines Cyclopropancarbonsäureesters mit Ammoniak in einem einwertigen Alkohol mit 1 bis 8 C-Atomen als Lösemittel, wobei die Reaktion durch ein Alkoholat mit 1 bis 8 C-Atomen katalysiert und bei 60 bis 200 °C durchgeführt wird.

Cyclopropancarbonsäureamid ist eine wichtige Feinchemikalie. Von großer Bedeutung ist z. B. seine Umwandlung in das Cyclopropanamin, welches im Pharma- und Pflanzenschutzbereich eingesetzt wird. Erforderlich sind daher Herstellungsverfahren von Cyclopropancarbonsäureamid, die eine hohe Reinheit des Produkts gewährleisten und gleichzeitig eine hohe Ausbeute ermöglichen.

Die Herstellung von Cyclopropancarbonsäureamid durch Amidierung von Cyclopropancarbonsäureestern ist bekannt.

So wird in US-A-3 711 549 Cyclopropancarbonsäuremethylester bei 80 °C mit Ammoniak unter Verwendung von 15 Mol-% einer 10- bis 17%igen Natriummethylatlösung in Methanol als Katalysator umgesetzt, wobei ein Zusatz von Toluol notwendig ist, um gute Amidausbeuten (Umsatz: ca. 90 %) zu erzielen. Die Reaktionszeit beträgt hier ca. 4 Stunden. Nach US-A-3 711 549 ist das Toluol notwendig, um das Gleichgewicht in die Richtung des Amids zu verschieben. Die in diesem Patent beschriebene Aufarbeitung ist sehr aufwendig. Nach der Reaktion werden nicht umgesetzter Cyclopropancarbonsäuremethylester, Toluol und Methanol abdestilliert. Das im Sumpf zurückbleibende Natriummethylat wird mit verdünnter Salzsäure neutralisiert und das Amid auf diese Weise in Wasser aufgenommen. Es resultiert daher eine stark verdünnte Lösung, die außerdem noch Natriumchlorid enthält.

In EP-A-0 205 403 wird angegeben, daß die Amidierung von Estern der Cyclopropancarbonsäure mit sterisch gehinderten sekundären oder tertiären Alkoholen in guten Ausbeuten gelingt, wenn als basischer Katalysator ein Natriumsalz eines mehrwertigen Alkohols, wie Glykol oder Glycerin, verwendet wird. Die Herstellung des Katalysators erfolgt hier, indem Natriumhydroxid in einem Überschuß des mehrwertigen Alkohols gelöst wird und das Reaktionswasser im Vakuum abgezogen wird. Für die Amidierung wird vorzugsweise 20 Mol-% Natriumglykolat verwendet. Um ein homogenes Reaktionsgemisch zu erhalten, wird ein großer Überschuß an Glykol oder der eines Kohlenwasserstoff-Lösemittels, wie z. B. Xylol, verwendet. Die Reaktion von Cyclopropancarbonsäureestern in diesem Medium bei einer Temperatur von etwa 100 °C und einem Ammoniakdruck von 3 bis 6 bar führt nach ca. 3 Stunden zu einem Umsatz von > 99 %. Die Aufarbeitung des Amids gestaltet sich jedoch schwierig. Das Reaktionsgemisch muß auf 0 °C gekühlt werden, damit genügend Produkt auskristallisiert. Anschließend wird das abfiltrierte Produkt mit Butanol gewaschen. Die Mutterlauge kann nach destillativer Entfernung des Butanols für eine erneute Amidierung eingesetzt werden. Nach 5 Reaktionszyklen erhält man eine durchschnittliche Amidausbeute von 92 %. Dieses Verfahren hat aber den Nachteil, daß mindestens 2 verschiedene Lösemittel verwendet werden und außerdem die Bereitung des Katalysators sehr aufwendig ist.

Eine weitere Vereinfachung der Herstellung wird in EP-A-0 365 970 beschrieben. Cyclopropancarbonsäureester der Formel 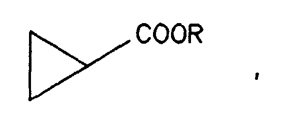 worin R geradkettiges oder verzweigtes C₄- bis C₈-Alkyl darstellt, werden dabei in Gegenwart eines Alkalimetallalkoholats eines einwertigen C₁- bis C₈-Alkohols umgesetzt. Dabei erhält man sehr gute Ausbeuten an Cyclopropancarbonsäureamid, wobei in dem Patent darauf hingewiesen wird, daß kein zusätzliches Kohlenwasserstoff-Lösemittel nötig ist. Das Produkt wird nach der Reaktion durch Filtration gewonnen. Die Mutterlauge kann, nachdem überschüssiges Butanol durch Destillation abgetrennt worden ist, weiterhin für mehrere Umsetzungen eingesetzt werden. Die Amidausbeute ist dann praktisch quantitativ.

Der Esterumsatz liegt in diesem Verfahren bei > 99 %, wobei das Reaktionsgemisch, aus dem das Amid auskristallisiert, noch rührfähig und gut filtrierbar ist. Das bei der Reaktion freiwerdende Butanol dient dazu, ein handhabbares Reaktionsgemisch zu erhalten, das dennoch konzentriert genug ist, um durch Filtration bei Raumtemperatur genügend Produkt zu erhalten.

Dieses Verfahren ist jedoch beschränkt auf den Einsatz von Cyclopropancarbonsäureestern höherer Alkohole (C₄ bis C₈), da nur bei der Verwendung dieser Ester der bei der Amidierung entstehende Alkohol dafür sorgt, daß auch nach einem 99%igen Umsatz das Reaktionsgemisch bei Raumtemperatur noch rührbar und gut filtrierbar bleibt.

Bei der Verwendung von Estern niederer Alkohole (C₁ bis C₃) resultiert - unter Verwendung von gleichen Alkoholatkonzentrationen - nach vollständigem Esterumsatz ein festes Reaktionsgemisch, das nicht mehr filtrierbar ist. Wenn aber eine stärker verdünnte Alkoholatlösung eingesetzt wird, sinkt die Reaktionsgeschwindigkeit der Amidierung drastisch.

Aufgabe der Erfindung ist es daher, Cyclopropancarbonsäureamid durch Amidierung von Estern der Cyclopropancarbonsäure mit kurzkettigen Alkoholen unter milden Bedingungen und ohne Verwendung von zusätzlichen Kohlenwasserstoff-Lösemitteln in hohen Ausbeuten herzustellen. Dabei sollte auch der einzusetzende Katalysator einfach zugänglich und möglichst kommerziell verfügbar sein.

Diese Aufgabe wird erf indungsgemäß dadurch gelöst, daß man Cyclopropancarbonsäureester der Formel 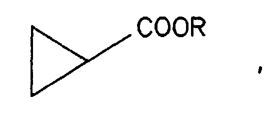 wobei R ein C₁- bis C₃-Alkyl oder Cyclopropyl ist, mit Ammoniak in alkoholischer Lösung, katalysiert durch ein Alkoholat eines einwertigen Alkohols mit 1 bis 8 C-Atomen, bei 60 bis 200 °C amidiert.

Führt man die Amidierung bis zu einem Umsatz von > 99 %, so resultiert ein bei Raumtemperatur festes Reaktionsprodukt, das bei der Aufarbeitung schwierig zu handhaben ist. Vorzugsweise wird deshalb bis zu einem Umsatz von 60 bis 90 % amidiert, ausgefallenes Cyclopropancarbonsäureamid abgetrennt und die verbleibende Mutterlauge einer erneuten Amidierung zugeführt.

Durch diese Maßnahmen gelingt es, Cyclopropancarbonsäureamid entgegen den Angaben in US 3 711 549 auch ohne Zusatz von z. B. Toluol in hohen Ausbeuten, d. h. in Ausbeuten von über 80 %, vorzugsweise von über 90 %, herzustellen.

Ein wesentlicher Aspekt der bevorzugten Ausführungsform der Erfindung ist es, daß die Amidierung von Cyclopropancarbonsäureestern niederer Alkohole nur bis zu einem Esterumsatz von 60 bis 90 %, vorzugsweise von 70 bis 85 %, durchgeführt wird, da die Mutterlauge für eine weitere Amidierung eingesetzt wird. Das resultierende Reaktionsgemisch ist dann auch bei Raumtemperatur noch rührbar. Das Gemisch kann problemlos filtriert oder zentrifugiert werden. Die Mutterlauge, die neben Alkoholat noch unumgesetzten Ester enthält, wird gegebenenfalls nach der Ergänzung des Esters für eine neue Amidierung eingesetzt.

Geeignete Ester der Cyclopropancarbonsäure sind beispielsweise die Methyl-, Ethyl-, Propyl- und Isopropylester. Bevorzugt werden die Methyl- und Ethylester der Cyclopropancarbonsäure eingesetzt, da die dann nach der Reaktion entstehenden Alkohole Methanol und Ethanol bei relativ niedrigen Temperaturen abdestilliert werden können.

Als Katalysatoren kommen Magnesium-, Calcium-, Barium- und vor allem Alkalialkoholate in Betracht. Dabei können die Alkoholate der Alkalimetalle Lithium, Natrium, Kalium, Rubidium oder Cäsium eingesetzt werden, wobei Natrium- und Kaliumalkoholate bevorzugt werden.

Es können Alkoholate von beispielsweise Methanol, Ethanol, Propanol, Isopropanol, n-Butanol, t-Butanol, Hexanol, Heptanol oder Octanol verwendet werden. Vorzugsweise setzt man jedoch Alkoholate von C₁- bis C₄-Alkoholen ein, wobei Alkalimethylat ganz besonders bevorzugt wird. Im allgemeinen besitzt dabei der Alkohol, der als Lösemittel eingesetzt wird, und der, von dem sich das Alkoholat herleitet, die gleiche Anzahl an C-Atomen.

Das Alkoholat wird vorzugsweise in einer Menge von 2 bis 40 Mol-%, im besonderen von 10 bis 30 Mol-%, bezogen auf die Molzahl des Cyclopropancarbonsäureesters, eingesetzt.

Wegen der guten Löslichkeit des Ammoniaks in Alkohol kann bei einem vergleichsweise niedrigen Ammoniakdruck amidiert werden. Die Amidierung wird vorzugsweise bei einem Ammoniakdruck von 1 bis 6 bar, besonders bevorzugt bei 1 bis 3 bar, durchgeführt. Ein besonderes Kennzeichen des erfindungsgemäßen Verfahrens ist, daß die Amidierung auch bei einem Ammoniakdruck von 1 bar durchgeführt werden kann und dabei das Amid in hohen Ausbeuten liefert.

Die Amidierung wird vorzugsweise bei 60 bis 150 °C ausgeführt, wobei Temperaturen von 60 bis 100 °C ganz besonders bevorzugt werden.

Bei der Abtrennung des Cyclopropancarbonsäureamids als Kristallisat, was im allgemeinen bei 0 bis 25 °C durch Filtration oder Zentrifugation erfolgt, werden die Bedingungen vorzugsweise so gewählt, daß die Sättigungskonzentration des Alkoholats im Gemisch nicht überschritten wird. Dadurch kann praktisch die Gesamtmenge an Alkoholat für eine erneute Amidierung wiederverwendet werden. Für diese zweite Amidierung wird vorzugsweise Cyclopropancarbonsäureester nachdosiert.

Ein wesentlicher Vorteil des Verfahrens ist, daß die Amidierung ohne ein zusätzliches Lösemittel, wie z. B. Toluol, Xylol, Ethylenglykol oder Glycerin, durchgeführt werden kann und trotzdem die Cyclopropancarbonsäureester niederer Alkohole verwendet werden können. Cyclopropancarbonsäureester niederer Alkohole (C₁ bis C₃) sind außerdem, beispielsweise gemäß DE-A-42 22 497, leicht herzustellen.

Der Einsatz dieser Ester erhöht auch die Raum-Zeit-Ausbeute der Amidierung. Die höchsten Raum-Zeit-Ausbeuten erzielt man dabei, wenn der Methyl- oder der Ethylester der Cyclopropancarbonsäure als Edukt dient. Die Wahl der Ester niederer Alkohole ermöglicht es, den bei der Amidierung entstehenden Alkohol auf einfache Weise bei niedriger Temperatur abzudestillieren.

Bei der praktischen Durchführung des erfindungsgemäßen Verfahrens geht man im allgemeinen so vor, daß man das Alkoholat in Form einer 10- bis 40%igen Lösung in dem entsprechenden Alkohol zu dem Cyclopropancarbonsäureester gibt. Nach Zusatz von Ammoniak wird im Autoklaven auf die Reaktionstemperatur erhitzt.

Nach der Reaktion wird das Produkt abfiltriert und mit Alkohol gewaschen. Die Mutterlauge wird eingeengt und kann nach Ergänzung des Esters für eine nächste Amidierung verwendet werden. Auf den Umsatz des Esters bezogen, ist die Ausbeute an Amid über mehrere Reaktionszyklen hinweg praktisch quantitativ.

Wahlweise kann nach der Amidierungsreaktion das Reaktionsgemisch für 2 Stunden auf 0 bis 5 °C gekühlt werden. Cyclopropancarbonsäureamid wird abfiltriert und mit Alkohol gewaschen. Die Mutterlauge wird eingeengt und kann ebenfalls wieder für eine Amidierung von Cyclopropancarbonsäureester eingesetzt werden. Auch hier ist die so erzielte Ausbeute an Amid, bezogen auf den Ester, nahezu quantitativ, wenn die Reaktion über mehrere Zyklen hinweg durchgeführt wird.

Die folgenden Beispiele sollen die Erfindung erläutern.

### Beispiel 1

### (vollständiger Esterumsatz ohne Wiedereinsatz der Mutterlauge)

In einem 5-l-Rührautoklaven werden 500 g (5 Mol) Cyclopropancarbonsäuremethylester und 90 g (0,5 Mol) 30%ige Natriummethylatlösung bei 70 °C vorgelegt. Bis zu einem Druck von 5 bar wird Ammoniak aufgedrückt. Dabei steigt die Temperatur innerhalb von 2 Minuten auf 80 °C an. Man läßt 5 Stunden bei 80 °C und einem Ammoniakdruck von 5 bis 5,5 bar reagieren. Dann läßt man abkühlen und drückt kein Ammoniak mehr nach, wobei der Druck auf etwa 2 bar fällt. Der feste Reaktorinhalt wird in 500 g Methanol aufgenommen. Durch GC-Analyse ergibt sich ein Esterumsatz von > 99 %. Die Lösung wird in einem Destillationskolben zur Trockne eingeengt. Der Rückstand wird aus heißem Methanol umkristallisiert und das Produkt mit kaltem Methanol gewaschen (312 g, Reinheit: 99,5 %, Ausbeute: 73 %). Die Mutterlauge (480 g) wird anschließend auf 182 g eingeengt und auf 5 °C gekühlt. Das ausgefallene Amid wird abfiltriert und mit kaltem Methanol gewaschen (49 g, Reinheit: 99,5 %, Ausbeute: 11,5 %).
Gesamtausbeute: 84,5 %

### Beispiel 2

### (vollständiger Esterumsatz ohne Wiedereinsatz der Mutterlauge)

Die Amidierung wird wie in Beispiel 1 durchgeführt (Esterumsatz: > 99 %). Der feste Reaktorinhalt wid mit 500 g Methanol aus dem Autoklaven herausgeholt. Zu dieser Lösung gibt man 242 g 20%ige Salzsäure (der pH-Wert der Lösung beträgt dann 7). Das Gemisch wird zur Trockne eingeengt und der Rückstand in Methanol aufgenommen.

Festes Natriumchlorid wird abfiltriert. Die Lösung wird nochmals zur Trockne eingeengt. Man nimmt erneut in Methanol auf und filtriert Natriumchloridreste ab. Die Methanol lösung wird dann auf 400 g eingeengt und auf 5 °C gekühlt. Das ausgefallene Amid wird abfiltriert und mit kaltem Methanol gewaschen (345 g, Reinheit: 99,5 %, Ausbeute: 81 %). Die Mutterlauge wird dann auf 100 g eingeengt und auf 5 °C gekühlt. Man filtriert das ausgefallene Amid ab und wäscht mit wenig kaltem Methanol (55 g, GC-Reinheit: 99,5 %, Ausbeute : 13 %). Gesamtausbeute: 94 %

### Beispiel 3

### (unvollständiger Esterumsatz mit Wiedereinsatz der Mutterlauge)

In einem 2-l-Vierhalskolben werden 700,7 g (7 Mol) Cyclopropancarbonsäuremethylester und 252,7 g (1,4 Mol) 30%ige Natriummethylatlösung bei 60 °C vorgelegt. Unter Rühren leitet man Ammoniak in das Gemisch ein. Es wird permanent soviel Ammoniak nachdosiert, daß sich ein Überdruck von etwa 1,1 bis 1,3 bar einstellt. Man läßt 14 Stunden bei 60 °C rühren. Nach dieser Zeit beträgt der Esterumsatz 71 %.

Man kühlt das Reaktionsgemisch auf 5 °C ab und filtriert das Amid nach 2 Stunden ab. Das Produkt wird mit kaltem Methanol gewaschen und getrocknet (280 g; 3,3 Mol; 47 %). Ein Teil des Methanols (200 g) wird aus der Mutterlauge herausdestilliert. Zu der verbleibenden Mutterlauge gibt man den Anteil Ester hinzu, der in dem ersten Ansatz umgesetzt worden ist (480 g; 4,89 Mol).

Man wärmt auf 60 °C auf und leitet für 12,5 Stunden Ammoniak ein (Esterumsatz: 81 %). Anschließend kühlt man auf 5 °C ab. Das ausgefallene Amid wird abfiltriert, mit Methanol gewaschen und getrocknet (450 g; 5,3 Mol; 93 %). Die Mutterlauge wird wieder eingeengt (abdestillierte Methanolmenge: 200 g). Die Ergebnisse zweier weiterer Reaktionszyklen lauten: Esterumsatz: 79 % bzw. 70 %; Ausbeute, bezogen auf Umsatz: 85 % bzw. 90 %.

### Beispiel 4

### (unvollständiger Esterumsatz mit Wiedereinsatz der Mutterlauge)

In einem 2-l-Vierhalskolben werden 700,7 g (7 Mol) Cyclopropancarbonsäuremethylester und 252,7 g (1,4 Mol) 30%ige Natriummethylatlösung bei 60 °C vorgelegt. Unter Rühren leitet man Ammoniak in das Gemisch ein. Es wird permanent soviel Ammoniak nachdosiert, daß sich ein Überdruck von 1,1 bis 1,3 bar einstellt. Man läßt 14 Stunden bei 60 °C rühren. Nach dieser Zeit beträgt der Esterumsatz ca. 70 %. Anschließend wird das bei der Amidierung entstandene Methanol (157 g) abdestilliert. Dabei steigt aufgrund des noch gelösten Ammoniaks der Umsatz auf ca. 80 %. Man läßt das Reaktionsgemisch auf Raumtemperatur abkühlen, filtriert das auskristallisierte Produkt ab und wäscht mit 100 g kaltem Methanol (0 bis 5 °C) nach. Ausbeute: 287 g (3,37 Mol; 60 %, bezogen auf den Umsatz)

Zur Mutterlauge gibt man 560 g (5,6 Mol) Cyclopropancarbonsäuremethylester und startet die Reaktion von neuem. Man setzt wieder bis zu einem Umsatz von 70 % um und destilliert dann das Reaktionsmethanol (157 g) ab, wobei ein Esterumsatz von 80 % resultiert. Die Amidausbeute beträgt dann 423 g (4,97 Mol; 89 %, bezogen auf den Esterumsatz).

## Patentansprüche

1. Verfahren zur Herstellung von Cyclopropancarbonsäureamid bei erhöhter Raum-Zeit-Ausbeute durch Amidierung von Cyclopropancarbonsäureestern mit Ammoniak in alkoholischer Lösung, katalysiert durch ein Alkoholat eines einwertigen Alkohols mit 1 bis 8 C-Atomen bei 60 bis 200 °C,
dadurch gekennzeichnet,
daß man einen Cyclopropancarbonsäureester der Formel 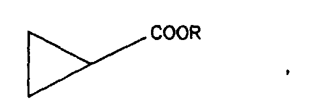 wobei R ein C₁- bis C₃-Alkyl oder Cyclopropyl ist,
- in Abwesenheit eines Kohlenwasserstoff-Lösemittels bis zu einem Umsatz von 60 bis 90 % amidiert,
- festes Cyclopropancarbonsäureamid abtrennt und
- die verbleibende Mutterlauge einer erneuten Amidierung zuführt.

2. Verfahren nach Anspruch 1,
dadurch gekennzeichnet,
daß man Cyclopropancarbonsäuremethylester oder Cyclocarbonsäureethylester einsetzt.

3. Verfahren nach Anspruch 1,
dadurch gekennzeichnet,
daß man mit einem Alkalialkoholat katalysiert.

4. Verfahren nach Anspruch 1,
dadurch gekennzeichnet,
daß der Alkohol 1 bis 4 C-Atome aufweist.

5. Verfahren nach Anspruch 1,
dadurch gekennzeichnet,
daß 2 bis 40 Mol-% Alkoholat, bezogen auf die Molzahl des Cyclopropancarbonsäureesters, eingesetzt werden.

6. Verfahren nach Anspruch 1,
dadurch gekennzeichnet,
daß bei einem Ammoniakdruck von 1 bis 6 bar, vorzugsweice von 1 bis 3 bar, gearbeitet wird.

7. Verfahren nach Anspruch 1,
dadurch gekennzeichnet,
daß die Amidierung bei 60 bis 150 °C, vorzugsweise bei 60 bis 100 °C durchgeführt wird.

8. Verfahren nach Anspruch 1,
dadurch gekennzeichnet,
daß der Cyclopropancarbonsäureester zu 70 bis 85% umgesetzt wird.

9. Verfahren nach Anspruch 1,
dadurch gekennzeichnet,
daß bei der Abtrennung des Cyclopropancarbonsäureamids als Kristallisat die Sättigungskonzentration des Alkoholats im Reaktionsgemisch nicht überschritten wird.

10. Verfahren nach Anspruch 1,
dadurch gekennzeichnet,
daß die Mutterlauge nach Ergänzung des Cyclopropancarbonsäureesters einer Amidierung zugeführt wird.

## Claims

1. A process for the preparation of cyclopropanecarboxamide at elevated space/time yield by amidating cyclopropanecarboxylic esters with ammonia in alcoholic solution, catalysed by an alcoholate of a monohydric alcohol of 1 to 8 carbon atoms, at from 60 to 200°C, characterized in that a cyclopropanecarboxylic ester of the formula 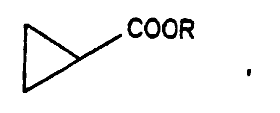 in which R is a C₁ to C₃ alkyl or cyclopropyl,
- is amidated in the absence of a hydrocarbon solvent up to a conversion of from 60 to 90%,
- solid cyclopropanecarboxamide is separated off, and
- the remaining mother liquor is fed to a further amidation reaction.

2. A process according to claim 1, characterized in that methyl cyclopropanecarboxylate or ethyl cyclopropanecarboxylate is employed.

3. A process according to claim 1, characterized in that the catalyst is an alkali metal alcoholate.

4. A process according to claim 1, characterized in that the alcohol has from 1 to 4 carbon atoms.

5. A process according to claim 1, characterized in that from 2 to 40 mol% of alcoholate are employed, based on the number of moles of the cyclopropanecarboxylic ester.

6. A process according to claim 1, characterized in that it is carried out under an ammonia pressure of from 1 to 6 bar, preferably from 1 to 3 bar.

7. A process according to claim 1, characterized in that the amidation is carried out at from 60 to 150°C, preferably from 60 to 100°C.

8. A process according to claim 1, characterized in that the cyclopropanecarboxylic ester is converted to the extent of from 70 to 85%.

9. A process according to claim 1, characterized in that, when the cyclopropanecarboxamide is separated off in the form of crystals, the saturation concentration of the alcoholate in the reaction mixture is not exceeded.

10. A process according to claim 1, characterized in that the mother liquor is supplied to an amidation reaction after supplementing the cyclopropanecarboxylic ester.

## Revendications

1. Procédé pour la préparation de cyclopropanecarboxamide avec un rendement espace-temps amélioré, par amidation d'esters d'acide cyclopropanecarboxylique en solution alcoolique avec de l'ammoniac, catalysée par l'alcoolate d'un alcool monovalent comportant de 1 à 8 atomes de carbone, à une température comprise entre 60 et 200 ° C, caractérisé en ce que
- l'on effectue l'amidation d'un cyclopropanecarboxylate de formule 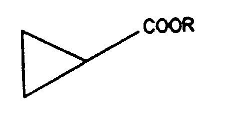 dans laquelle R est un résidu alkyle en C₁₋₃ ou un résidu cyclopropyle, en absence d'un solvant hydrocarboné jusqu'à un avancement de la réaction de 60 - 90 %,
- en ce que l'on sépare le cyclopropanecarboxamide solide, et
- en ce que l'on utilise la liqueur-mère résiduelle pour une nouvelle réaction d'amidation.

2. Procédé conforme à la revendication 1, caractérisé en ce que l'on utilise le cyclopropanecarboxylate de méthyle ou le cyclopropanecarboxylate d'éthyle.

3. Procédé conforme à la revendication 1, caractérisé en ce que l'on utilise, comme catalyseur, un alcoolate de métal alcalin.

4. Procédé conforme à la revendication 1, caractérisé en ce que l'alcool comporte de 1 à 4 atomes de carbone.

5. Procédé conforme à la revendication 1, caractérisé en ce que l'on utilise de 2 à 40 % en moles d'alcoolate, rapporté au nombre de moles de cyclopropanecarboxylate.

6. Procédé conforme à la revendication 1, caractérisé en ce que l'on opère à une pression d'ammoniac comprise entre 1 et 6 bars, de préférence entre 1 et 3 bars.

7. Procédé conforme à la revendication 1, caractérisé en ce que l'on effectue l'amidation à une température comprise entre 60 et 150 °C, de préférence entre 60 et 100 °C.

8. Procédé conforme à la revendication 1, caractérisé en ce que le degré de transformation du cyclopropanecarboxylate est compris entre 70 et 85 %.

9. Procédé conforme à la revendication 1, caractérisé en ce que, lors de la séparation du cyclopropanecarboxamide sous forme de produit de cristallisation, on ne dépasse pas la concentration de saturation de l'alcoolate dans le mélange réactionnel.

10. Procédé conforme à la revendication 1, caractérisé en ce que la liqueur-mère, après avoir reçu un complément de cyclopropanecarboxylate, est soumise de nouveau à une amidation.
